# EUROPEAN PATENT APPLICATION

(11) **EP 1 762 556 A1**
(43) Date of publication of application: **14.03.2007**
(21) Application number: 05104321.4
(22) Date of filing: 20.05.2005
(51) Int. Cl.: C07C 29/62, C07C 31/36, C07C 29/82

(54) **Process for producing dichloropropanol from glycerol**

(71) Applicant: SOLVAY (Société Anonyme), 1050 Bruxelles (BE)
(72) Inventor: Gilbeau, Patrick, 7090, Braine-le-Comte (BE); Andolenko, Ivan, 39500, Tavaux (FR); Krafft, Philippe, 1640, Rhode Saint Genese (BE); Gielen, Freddy, 1330 Rixensart (BE)
(74) Representative: Jacques, Philippe

(57) **Abstract**

Process for producing dichloropropanol, according to which glycerol containing metal salt is subjected to a reaction with a chlorinating agent in a reaction mixture. At least a fraction obtained from the reaction mixture, which fraction contains metal salt, is subjected to a treatment comprising one or more separation operations to remove metal salt from said fraction.

## Description

The present application is related to PCT application No. EP 2004/053008 in the name of the Applicant, the entire contents and preferences of which are incorporated herein by reference.

The present invention relates to processes for producing an organic compound, in particular to a process for producing dichloropropanol.

It is known that natural petrochemical resources, for example oil or natural gas, that are available on earth are limited. Now, these resources are used for producing fuels and as a starting product for producing a large variety of useful organic compounds such as monomers or reactants for producing plastics, for example epichlorohydrin or dichloropropanol (see, for example, Ullmann's Encyclopedia of Industrial Chemistry, 5. ed., Vol. A9, p. 539-540). Documents Chemistry and Industry, November 20, 1931, Part III, pages 949 to 954, and November 27, 1931, Part III , pages 970 to 975, describe a process for the synthesis of dichloropropanol from glycerol and hydrochloric acid in the presence of acetic acid as acid catalyst.

According to known processes for producing dichloropropanol, the product is generally obtained in highly diluted solution with a titre of 5 to 15% by weight. It is then particularly expensive to purify it. Moreover, the major isomer obtained according to such processes is 2,3-dichloropropane-1-ol.

It was desirable to fmd uses and processes making it possible to reduce the consumption of natural petrochemical resources, in particular for the abovementioned uses.

It was also desirable to find processes for re-using by-products of other production processes so as to minimize the overall amount of by-products having to be eliminated or destroyed.

Consequently, the invention relates to the use of glycerol obtained from renewable raw materials, as a starting product for producing organic compounds.

The expression "glycerol obtained from renewable raw materials" is intended to denote in particular glycerol obtained in the course of the production of biodiesel, or else glycerol obtained during conversions of fats or oils of plant or animal origin in general, such as saponification, trans-esterification or hydrolysis reactions. A particularly suitable glycerol can be obtained during the conversion of animal fats. Another particularly suitable glycerol can be obtained during the production of biodiesel.

In contrast, synthetic glycerol is generally obtained from petrochemical resources.

In the use according to the invention, the glycerol can be a crude product or a purified product. When the glycerol is a crude product, it can comprise, for example, water and a metal salt, in particular a metal chloride, which is preferably chosen from NaCl and KCl. The metal salt can also be selected from metal sulphates such as sodium sulphate and potassium sulphate. The crude product can also contain organic impurities such as carbonyl compounds, in particular aldehydes, fatty acids, or esters of fatty acids, such as in particular monoglycerides or diglycerides, optionally in combination with water and/or the metal chloride.

In the use according to the invention, the crude product generally comprises at most 8 % by weight of organic impurities. Often, the crude product comprises at most 6 % by weight of organic impurities. Preferably, it comprises at most 2 % by weight of organic impurities. Most preferably, it comprises at most 1 % by weight of organic impurities. The organic impurities typically consist essentially of fatty acids and their derivatives.

It has surprisingly been found that the use of crude product having a high content of organic impurities does not have substantial impact on the reaction underlying the process of the invention. Optional byproducts from the organic impurities can easily be eliminated from the reaction mixture e.g., if applicable, by controlling the purge rate.

In the use according to the invention, the crude product generally comprises at least 40% by weight of glycerol. Often, the crude product comprises at least 50% by weight of glycerol. Preferably, it comprises at least 70% by weight of glycerol. Often, the crude product comprises at most 99% by weight of glycerol. Typically, it comprises at most 95% by weight of glycerol.

In the use according to the invention, the crude product generally comprises at least 5% by weight of water or, in the absence of other compounds, at least 1% by weight of water. In the use according to the invention, the crude product generally comprises at most 50% by weight of water or, in the absence of other compounds, at most 60% by weight of water. Often, the crude product comprises at most 30% by weight of water, preferably at most 21% by weight of water.

In another embodiment, the crude product comprises at most 89% by weight of glycerol. In that embodiment, the crude product comprises at most 85% by weight of glycerol. In that embodiment, the crude product comprises generally at least 10% by weight of water and often at least 14% by weight of water.

Where appropriate, the crude product generally has a metal salt, in particular a metal chloride content of at least 1% by weight, preferably greater than or equal to approximately 3% by weight. Where appropriate, the crude product generally has a metal salt, in particular a metal chloride content of at most 10% by weight, preferably less than or equal to approximately 5% by weight.

The use according to the invention applies particularly preferably to the production of chlorinated compounds such as dichloropropanol and epichlorohydrin. Surprisingly, the use according to the invention makes it possible to economically obtain these compounds starting from renewable resources.

Consequently, the invention also relates to a process for producing an organic compound, comprising the use according to the invention.

Consequently, the invention also relates in particular to a method for producing a chlorinated organic compound, according to which glycerol obtained from renewable raw materials is used, in accordance with the use according to the invention, and said glycerol is brought into contact with at least one chlorinating agent. It is understood that the methods of production described hereinafter can also be carried out with glycerol in general and are not limited to the preferred use of glycerol obtained from renewable raw materials.

In the process for producing a chlorinated organic compound according to the invention, the chlorinating agent is as disclosed specifically in PCT EP 2004/053008

Mention may in particular be made of a chlorinating agent comprising hydrogen chloride.

In the process for producing a chlorinated organic compound according to the invention. It is preferred to carry out the reaction in the presence of a catalyst, as specifically disclosed in PCT EP 2004/053008.

Mention may in particular be made of a catalyst based on a carboxylic acid or acid derivative having an atmospheric boiling point of greater than or equal to 200°C, in particular adipic acid and adipic acid derivatives.

In the process according to the invention, the reaction is generally carried out at temperature, pressure and residence time as specifically disclosed in PCT EP 2004/053008

In the process for producing a chlorinated organic compound according to the invention, at least dichloropropanol is preferably obtained as chlorinated organic compound.

The term "dichloropropanol" is generally intended to mean a mixture of isomers consisting essentially of 1,3-dichloropropane-2-ol and of 2,3-dichloropropane-1-ol.

In the process for producing a chlorinated organic compound according to the invention, a high selectivity for 1,3-dichloropropane-2-ol is surprisingly obtained, which isomer is particularly suitable as starting product for a dehydrochlorination with a view to producing epichlorohydrin.

In another variant of the process for producing a chlorinated organic compound according to the invention, vapour stripping, in particular steam stripping of the reaction medium, is carried out. In this case, it is possible to obtain a fraction containing from 1 to 5, some times from 2 to 3 and preferably from 1.5 to 2.5 mol/l of chlorinated organic compound, in particular of dichloropropanol. In this variant, the stripped mixture is mainly composed of water and dichloropropanol.

In a particular embodiment of the process according to the invention, continuous or periodic withdrawal of a fraction comprising at least water and chlorinated organic compound, in particular dichloropropanol, is carried out. Said fraction may also contain hydrogen chloride. Preferably, the fraction is withdrawn continuously as its constituents form. The fraction obtained can subsequently be subjected to an operation of separation by settling out.

In a particular variant, which is preferred when the reaction is carried out continuously and continuous or periodic withdrawal from the reaction of a fraction comprising at least water and chlorinated organic compound is carried out, the reaction medium is fed with water, in particular with steam. The feeding can be effected with extrinsic water originating from a suitable feed pipe or, optionally, with residual water recovered from another unit reaction or operation.

This feed is generally effected in such as way as to maintain the water concentration in the reaction medium within the ranges indicated above.

The variants involving continuous or periodic withdrawal can be effected by introducing into a distillation step a gaseous phase withdrawn starting from the reaction medium, in particular withdrawing and introducing into a distillation step a gas phase which is in equilibrium with a liquid reaction medium.. Where appropriate, this embodiment can be carried out in a reactor surmounted by a suitable distillation column. This embodiment is particularly suitable when aqueous hydrochloric acid is used as chlorinating agent. It is also possible to arrange a distillation column separated from the reactor, the liquid bottom of which can be sent back to the reaction medium. This embodiment is particularly suitable when hydrogen chloride, for example gaseous or essentially anhydrous hydrogen chloride, is used as chlorinating agent.

In this embodiment, the operating conditions of the reactor such as feed rates of reactants, in particular hydrogen chloride and glycerol, catalyst feed rate, temperature, reactor volume and pressure are preferably adjusted in such a way that the concentration of hydrogen chloride in the mixture fed in distillation remains lower than the concentration of hydrogen chloride in the binary azeotropic hydrogen chloride-water mixture at the pressure of the reaction. An effective means of adjusting this concentration is controlling the hydrogen chloride supply to the liquid reaction medium.

This embodiment of the process is preferably carried out continuously.

In one aspect, the fraction to be introduced into the distillation column separated from the reactor is withdrawn continuously or periodically, preferably continuously, from the liquid reaction medium and at least water and chlorinated organic compound is separated. In the distillation step, a fraction containing chlorinated organic compound may also be separated. In addition, one or more fractions containing organic products such as heavy byproducts and in particular catalyst and/or hydrogen chloride can also be separated in this distillation step and generally recycled to the reaction medium. By selecting an appropriate reflux ratio, it is possible to separate in this aspect a fraction containing at least water which is substantially free of hydrogen chloride.

The reflux ratio can suitably be adjusted by supplying water which is preferably substantially free of hydrogen chloride to the distillation column. In this embodiment, the water is preferably fed to the top of the distillation column, Water can be supplied, for example by recycling at least a portion of water separated in the distillation operation to the top of the distillation column. Water can also be supplied by adding fresh water to the top of the distillation column. Both manners of supplying water can be combined. Adding fresh water gives particularly good results.

"Substantially free of hydrogen chloride", is understood to denote in particular a hydrogen chloride content in the fraction comprising water equal to or less than 10% by weight relative to the total weight of the fraction comprising water. Often, this content is equal to less than 5% by weight and preferably equal to or less than 1% by weight and more preferably equal to or less than 0.3% by weight. If hydrogen chloride is present in the fraction "substantially free of hydrogen chloride" its content is generally equal to or more than 1 mg/kg, often equal to or more than 10 mg/kg relative to the total weight of the fraction comprising water.

"Fresh" water is understood to denote water having a content of constituents other than water, organic or inorganic, lower than or equal to 12 % by weight relative to the total weight of the water and of such constituents, preferably lower than or equal to 10 % by weight and most preferably lower than or equal to 1 % by weight. Generally, "fresh" water is understood to denote in particular water having a content of constituents other than water, organic or inorganic, equal to or more than 1 mg/kg, often equal to or more than 10 mg/kg relative to the total weight of water and of such constituents. A possible source of fresh water can be the water used for eluting metal salt as described herein below.

By constituents other than water, one intends to designate more specifically dichloropropanol.

It has been found that the exploitation of the liquid-vapour equilibrium properties of the water-hydrogen chloride-dichloropropanol ternary composition makes it possible to withdraw from the production reaction the reaction products comprising in particular dichloropropanol and water, while at the same time allowing most of the catalyst(s) and of the reactants (including the hydrogen chloride), to be recycled to the reactor.

In a preferred embodiment, a separation of a fraction of the reaction medium is carried out by a distillation step and the sum of materials fed to this distillation step has a hydrogen chloride concentration which is lower than the hydrogen chloride concentration in the binary azeotropic composition hydrogen chloride/water at the pressure of the distillation.

In consequence, the invention concerns also a process for separation of a mixture containing at least water, dichloropropanol and hydrogen chloride wherein the mixture is separated in a distillation step wherein the sum of materials fed to said distillation step has a hydrogen chloride concentration which is lower than the hydrogen chloride concentration in the binary azeotropic composition hydrogen chloride/water at the pressure of the distillation.

It is possible for example to control the hydrogen chloride content in the sum of materials fed to the distillation step by adding water. Such addition can be carried out for example by injection of vapor into the boiler of a distillation column used in the distillation step or by recycling to the distillation step of a water phase which can be obtained for example by decantation of a fraction withdrawn from the top of a distillation column, or by adding fresh water to the top of a distillation column or by adding a mixture of recycled and fresh water.

The maximum suitable hydrogen chloride concentration decreases slightly when the operating pressure is higher in agreement with the liquid-vapour equilibrium data for the azeotropic hydrogen chloride published by Bonner and Titus (J. Amer. Chem. Soc. 52, 633 (1930)) and partially reprinted in the Table hereafter :

| Pressure (Torr) | Temperature (°C) | HCl in azeotrope (% wt) |
|---|---|---|
| 50 | 48.74 | 23.42 |
| 250 | 85.21 | 21.88 |
| 370 | 90.24 | 21.37 |
| 540 | 99.65 | 20.92 |
| 760 | 108.58 | 20.22 |
| 1000 | 116.19 | 19.73 |
| 1220 | 122.98 | 19.36 |

In such conditions, a fraction comprising water which fraction is substantially free of hydrogen chloride as defined above can be recovered by distillation from the reaction mixture or the gas phase above the liquid reaction mixture, e.g. by distilling material withdrawn from said gas phase and obtaining the fraction comprising water preferably at the top of the distillation column.

For instance, at atmospheric pressure (101,3 kPa), it is possible to obtain by distillation of the reactor gas phase a binary azeotropic mixture of water and dichloropropanol containing 23 % by weight of dichloropropanol if the hydrogen chloride concentration in that gas phase in contact with the reaction medium is lower than about 20.22 % by weight.

When dichloropropanol is not completely removed from the reaction medium by withdrawal of a fraction containing water, it is possible to recover at least a fraction of the reaction medium containing dichloropropanol.

In this aspect of the process for producing a chlorinated organic compound according to the invention, at least one fraction comprising from 50 to 95% by weight of dichloropropanol and at most 50% by weight of water is generally recovered. Preferably, this fraction comprises from 75 to 99.9%, often from 75 to 99%, by weight of dichloropropanol and from 0.01 to 25%, often from 1 to 25%, by weight of water.

The recovery is preferably carried out by distillation or evaporation. Other fractions obtained during this step, comprising, for example, monochloropropanediol and, optionally, glycerol and catalyst, can be recycled to the reaction with the chlorinating agent. It is also possible to separate at least one fraction containing heavy by-products of the reaction, such as described above, in particular chlorinated polyglycerols, which can be destroyed or can optionally be used in a process for producing polyglycerols, for example by dechlorination.

The distillation or evaporation is generally carried out at a temperature of at least 20°C. This temperature is often at least 60°C. It is preferably at least 70°C. The distillation or evaporation is generally carried out a temperature of at most 180°C. This temperature is preferably at most 140°C.

The distillation or evaporation is generally carried out at a pressure of greater than 0.001 bar. This pressure is preferably greater than or equal to approximately 0.003 bar. The distillation or evaporation is generally carried out at a pressure of at most 1 bar. This pressure is often at most 0.5 bar. It is preferably at most 0.2 bar.

The distillation or evaporation operation can be carried out either by means of distillation columns or by means of evaporators, of film evaporators or alternatively of wiped thin film evaporators. The recoverable fractions of the residues can be separated therefrom advantageously by means of a wiped thin film evaporator with an interior or exterior condenser.

In a particular variant, the dichloropropanol is produced according to a process comprising:
(a) a first reaction step in which glycerol is brought into contact with the chlorinating agent so as to obtain a fraction of products comprising at least chloropropanediol;
(b) optionally at least part of the fraction of products is subjected to a drying operation;
(c) at least part of the fraction of optionally dried products is introduced into a second reaction step in which at least part of the chloropropanediol is reacted with the chlorinating agent.

Steps (a) and (c) in this variant are preferably carried out under conditions and with the preferences as described above for the process for producing a chlorinated organic compound according to the invention. However, it is preferred to carry out the reaction of step (a) in the presence of water at a concentration preferably ranging from 20 to 80% by weight relative to the total weight of the reaction medium.

Step (b) can be carried out, for example, by a stripping operation in at least one of the reactors of steps (a) or (c) or by means of an evaporator placed on a recirculation pipe exterior to the reactor. According to another preferred variant, the water is removed by means of a membrane technique.

The process for producing an organic compound, in particular dichloropropanol, according to the invention can be carried out, for example, in cascade reactors, in at least one plate column or in at least one bubble column, or an assembly of such reactors.

The reactors may effectively be of a type that is stirred either by means of internal stirring, or by means of a recirculation pipe exterior to the reactor.

When, in the process according to the invention, the reaction medium is heated, the heating can be obtained, for example, by means of a jacket or by means of an internal heat exchanger. Heating can also be obtained by means of a heat exchanger on a recirculation pipe exterior to the reactor. Optionally, the heating is obtained by combined use of a jacket and of a heat exchanger on a recirculation pipe exterior to the reactor.

In particular when the process according to the invention is operated in a continuous or fed-batch mode, secondary reactions can lead to the build-up in the reactor of by-products of low volatility, among which more or less chlorinated glycerol oligomers. This build-up can lead to a progressive increase of the volume of the reaction medium and require a continuous or discontinuous purge of the reactor to keep the volume at an adequate level.

If appropriate, the catalyst quantity which is removed during such purging operation can be compensated by the introduction of an equivalent quantity of pure or purified catalyst.

The catalyst contained in the purge from the reaction mixture can be economically recycled in the reactor after a purification treatment. For example, catalysts with low solubility in water can be subjected to an acid hydrolysis treatment, preferably carried out at a temperature higher than 30°C, preferably at least 50°C which is followed by a separation step e.g. by decantation, filtration or extraction. It has been found that in the case of adipic acid, an acid hydrolysis of the purge leads after cooling and filtration, to the recovery of cristallised adipic acid of high purity with a good yield.

In particular when the process according to the invention is operated in a continuous or fed-batch mode, metal salts, in particular NaCl, optionally present in the raw materials, for example in the glycerol from renewable resources described above, can concentrate in the reactor. An increase of metal salt content could possibly lead to a progressive crystallisation of insoluble materials, leading to an increase of the volume of the reaction medium and to various problems linked to the presence of solid materials such as deposit formation on the reactor walls, on the stirrer and on feed and purge lines and valves. Deposit formation on the reactor wall can reduce the heat transfer efficiency and require an increase amount of energy to maintain the temperature of the reaction medium. Deposit formation on valves and lines can lead to plugging problems. An increased amount of solid in the reaction mixture can reduce the stirring efficiency and require a higher amount of energy to reach a correct agitation. Increase of metal salt concentration could then require a higher continuous or discontinuous purge rate leading to higher losses of products.

While the presence of metal salt is surprisingly acceptable in the process according to the invention, it may therefore be desirable to remove metal salt, in particular NaCl, from the reaction system, e.g. in order to prevent optional accumulation of metal salt in the reaction mixture. Such removal can suitably be carried out by subjecting at least a fraction obtained from the reaction mixture which fraction contains metal salt to a treatment comprising one or more separation operations such as extraction, settling out, filtration and centrifugation. Another possible separation operation is a treatment with a resin. A filtration gives good results.

The fraction obtained from the reaction mixture can be obtained by directly withdrawing a portion of the reaction mixture, notably when the reaction is carried out in the liquid phase. The fraction obtained from the reaction mixture can also be obtained by withdrawing a portion of the reaction mixture and further treating said portion before removing the metal salt. An example of a suitable treatment is a concentration operation carried out on a liquid portion of reaction mixture wherein volatile compounds such as starting materials and products of the reaction, which may optionally be recovered and/or recycled to the reaction mixture, are separated e.g. by distillation or evaporation and a concentrated fraction having increased content of metal salt is obtained and subjected to the treatment to separate metal salt.

In a preferred embodiment, the purge of the reactor is sent to a least one separation unit, where the separation of the metal salt is carried out for example by extraction, settling out, filtration or centrifugation. A liquid/solid separation unit is preferred and a separation by filtration is more preferred. The separated liquid is preferably recycled back to the reactor and the metal salt is left on the filter.

The filtration step can be carried out at a temperature which is usually greater than or equal to 20 °C, preferably greater than or equal to 50 °C and most preferably greater than or equal to 80 °C. This temperature is generally lower than or equal to 150 °C and preferably lower than or equal to 140 °C.

The nature of the filtration system is not critical and is readily apparent to the skilled person aware of the present invention.

As the metal salt accumulates on the filtration system, it is generally recommended to periodically regenerate the filtration unit by removing the filtrated salt. The regeneration can be performed by any means, for example by removing, in particular by mechanical means, the solid or by dissolving the solid. Optionally, solid elution treatments can be incorporated in the regeneration procedure.

In a one embodiment according to the invention, the metal salt is removed as a solid from the filtration system without any pretreatment.

In a first variant, the salt is disposed off in a suitable manner without further treatment.

In a second variant, the salt is stored in a separate vessel for further treatment. Further treatment can include elution of the solid with solvents and dissolution of the solid with solvents. Such treatments are described herebelow in the preferred embodiment.

In a preferred embodiment according to the invention, the metal salt is treated before removal from the filtration system.

Optionally adsorbed products and reactants such as in particular catalyst, monochlorohydrins and dichloropropanols can be recovered from the metal salts, in particular from NaCl. for example by elution with an appropriate eluting solvent such as a mixture of water and dichloropropanol. It is particularly preferred to use a dichloropropanol saturated with water at room temperature. The water content of the dichloropropanol used as eluting solvent is generally lower than or equal to 20 % by weight and preferably lower than or equal to 15 % and most preferably lower than or equal to about 12 %. The water content in the mixture of water and dichloropropanol is generally higher than or equal to 1 % by weight.

In another embodiment, the eluting solvent consists essentially of dichloropropanol. In this embodiment, the water content is generally lower than 1 % by weight, preferably lower than or equal to 0.5 % by weight.

In still another embodiment, the eluting solvent is water for example fresh water as defined above.

The elution step can be carried out at a temperature which is usually greater than or equal to 20 °C, preferably greater than or equal to 50 °C and most preferably greater than or equal to 80 °C. This temperature is generally lower than or equal to 150 °C and preferably lower than or equal to 140 °C.

After elution, the solvent used for eluting the metal salt can be recycled to the hydrochlorination reactor.

In particular after elution with dichloropropanol the metal salt can then be optionally further eluted with an aqueous solution. The aqueous solution can arise from any step of the process. It is preferred to use fresh water as defined above.

The elution step can be carried out at a temperature which is usually greater than or equal to 20 °C, preferably greater than or equal to 50 °C and most preferably greater than or equal to 80 °C. This temperature is generally lower than or equal to 150 °C and preferably lower than or equal to 140 °C.

After elution, the aqueous solution used for eluting the metal salt can be sent to the hydrochlorination reactor, to a dehydrochlorination unit, to a biological treatment unit or to an oxidation treatment unit.

In a first variant, after elution with dichloropropanol and water, the salt is removed as a solid in a suitable manner without further treatment. The salt is then disposed off in a suitable manner.

In a second variant, after elution with dichloropropanol and water, the salt is dissolved with an aqueous solution.

The aqueous solution can arise from any step of the process. It is preferred to use fresh water as defined above.

The dissolution step can be carried out at a temperature which is usually greater than or equal to 20 °C, preferably greater than or equal to 50 °C and most preferably greater than or equal to 80 °C. This temperature is generally lower than or equal to 150 °C and preferably lower than or equal to 140 °C.

The aqueous solution containing the dissolved metal salt can be disposed off. Preferably, it is sent to a dehydrochlorination unit, to a biological treatment unit or to an oxidation treatment unit.

In the above variants, the elution of the metal salt with water and the dissolution of the metal salt with water can be part of a single unit operation.

The above operations are particularly suited when the metal salt is sodium chloride or potassium chloride and more particularly suited for sodium chloride.

When the purge is carried out in a discontinuous mode, one filtration unit is usually sufficient since the filtration system can be regenerated during the shut-downs of the purge. When the purge is carried out in a continuous mode, it is preferred to have at least two filtration units working in alternance, one being in filtration mode while the other is in regeneration mode.

When anhydrous HCl is used, it is preferred to direct a liquid stream comprising the glycerol against the current of the stream of HCl. When the process is carried out in several reactors, the HCl is advantageously dried between two reactors, for example by adsorption on a suitable solid, such as a molecular sieve, or by reverse osmosis through a suitable membrane.

This particular embodiment of the process according to the invention makes it possible to obtain, particularly economically, concentrated dichloropropanol often having a dichloropropanol content of greater than or equal to 90% by weight relative to the total weight of the dichloropropanol. By means of this approach, it is possible to obtain 1,3-dichloropropane-2-ol as major isomer with an isomeric purity of greater than 80%.

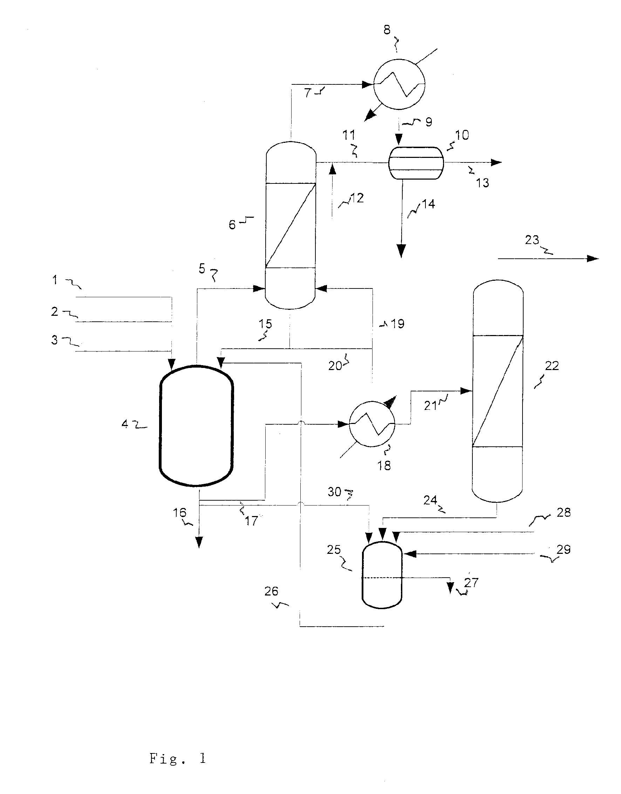

Figure 1 shows a preferred particular scheme for a plant that can be used for carrying out the process for producing dichloropropanol according to the invention : A reactor (4) is fed, in a continuous or batch mode, with glycerol via line (1) and catalyst via line (2), the feed of hydrogen chloride, anhydrous or in aqueous solution, is carried out continuously or in batch-mode via line (3), a distillation column (6) is fed via line (5) with vapour produced from reactor (4), a stream is withdrawn from column (6) via line (7) and fed to a condenser (8), the stream from the condenser is fed via line (9) to a decanter (10) in which aqueous and organic phases are separated. A fraction of the separated aqueous phase is optionally recycled via line (11) to the top of the column for maintaining reflux. Fresh water can be added via line (12) to the top of the column for maintaining reflux. The production of dichloropropanol is distributed between the organic phase withdrawn through line (14) and the aqueous phase withdrawn through line (13). The residue from column (6) can be recycled to the reactor via line (15). Heavy by-products can optionally be removed from the reactor by means of a purge (16) located in the liquid bottom of the reactor. A stream is withdrawn from the purge (16) and fed via line (17) into a stripper (18) wherein a partial stripping operation is carried out e.g. by heating or by gas sweeping with nitrogen or steam, the gas phase containing most of the hydrogen chloride from stream (17) is recycled via line (19) to the column (6) or via line (20) to the reactor (4), a distillation column or stripping column (22) is fed with the liquid phase arising from the stripper (18) via line (21), the main fraction of dichloropropanols is collected from the top of the column (22) through line (23) and the column residue is fed via line (24) to a filtration column (25) in which solid and liquid phases are separated, the liquid phase is recycled via line (26) to the reactor (4). The solid can be withdrawn from the filtration unit (25) via line (27) as a solid or as a solution. Solvents can be added to the filtration unit (25) via lines (28) and (29) for washing and/or dissolution of the solid and withdrawn from line (27). Optionally, a stream is withdrawn from the purge (16) and fed via line (30) into a filtration column (25). The stripper (18) and the distillation column (22) are then bypassed.

Results obtained according to this last scheme (stripper (18) and column (22) bypassed) are detailed in example 1.

This variant of the process allows to remove at the top by azeotropy almost all of the water arising from the reaction, from the starting materials and/or possibly fed in the bottom of the reactor or of the column and to obtain a mixture of dichloropropanols of very high purity, above 99.5 % by weight for the sum of the two isomers, with a selectivity related to hydrocarbon chain and hydrogen chloride higher than 99 % by weight and to remove the metal salt which can build up in the reactor when crude glycerol is used in the reaction.

The example below are intended to illustrate the invention without, however, limiting it.

### Example 1

The numbers in parentheses refer to Figure 1. The additional equipment in the schema of figure 1, with stripper (18) and column (22) has not been used in this case. Reactor (4) has been continuously fed with crude glycerol and a 33 % by weight hydrochloric aqueous acid solution with relative flow rates mass ratios of 2.06. The crude glycerol was a by product of the biodiesel production and contained 85 % of glycerol, 6 % of NaCl and 0.5 % of organic impurities (fatty acids and derivatives). The residence time was 16 h, the adipic acid concentration in the reaction medium was 2.5 mol of acid functionalities/kg. The reactor has been operated at atmospheric pressure and at 115°C. The reaction mixture has been stripped with of nitrogen and the generated vapor phase has been treated in the distillation column (6) via line (5) (figure 1). The gas phase removed from column (6) has been condensed at 25°C (8) and decanted in the decanter (10). Reflux ratio was adjusted to withdraw the entire production of dichloropropanol at the top of column by recycling an appropriate amount of the aqueous phase from the decantor. At the outlet of the decantor an aqueous phase containing 15.0 % of dichloropropanol (13) and an organic phase (14) containing 88 % of dichloropropanol were recovered. The profiles in organic impurities in these phases were not different from those observed when pure glycerol is used in the process.

A slurry from the reactor has been pumped on a 115 micrometer PTFE membrane filter in the filtration column (25). The salt cake in the filter has been washed at 20°C with dichloropropanol saturated with water. After removal of the liquid phase and draining of the solid, the salt has been dissolved in water and the salted water phase has been discarded. The duration of washing and salt dissolution was about 2 hours. A new filtration cycle of the slurry from the reactor has then been operated. The dichloropropanol washing has been recycled to the reactor by continuous feeding. The analysis of the water phase with salt indicated a dichloropropanol : NaCl mass ratio of 1.44 and a small amount of catalyst (less than 10 g/kg). The quantity of dichloropropanol in the salted water represented 1.6 % of the dichloropropanol total production.

The global yield in dichloropropanol was 93 %.

## Claims

1. Process for producing dichloropropanol, according to which glycerol containing metal salt is subjected to a reaction with a chlorinating agent in a reaction mixture, and wherein at least a fraction obtained from the reaction mixture, which fraction contains metal salt, is subjected to a treatment comprising at least one separation operation to remove metal salt from said fraction.

2. Process according to claim 1, wherein the metal salt, is selected from sodium chloride, potassium chloride, sodium sulfate and potassium sulfate.

3. Process according to claim 1 or 2 wherein the glycerol comprises from 1 to 10 % by weight of metal salt

4. Process according to claims 1 to 3 wherein the chlorinating agent is selected comprises hydrogen chloride

5. Process according to claims 1 to 4 wherein the reaction is carried out in the presence of a catalyst.

6. Process according to claim 5 wherein the catalyst is a carboxylic acid or a carboxylic acid derivative having an atmospheric boiling point of greater than or equal to 200°C, is preferably adipic acid and an adipic acid derivative.

7. Process according to claims 1 to 6 wherein the separation operation is selected from distillation, extraction, decantation, centrifugation, filtration and treatment with ion exchanged resins.

8. Process according to claim 7 wherein the separation operation is a liquid/solid separation and preferably a filtration operation and wherein the metal salt is removed as a solid.

9. Process for producing dichloropropanol, according to which glycerol containing at most 6 % by weight of organic impurities is subjected to a reaction with a chlorinating agent.

10. Process for producing dichloropropanol, according to which
(a) glycerol is subjected to a reaction with a chlorinating agent in a reaction mixture
(b) continuous or periodic withdrawal from the reaction mixture of a fraction comprising at least water and chlorinated organic compound, in particular dichloropropanol, is carried out;
(c) at least part of the fraction obtained in step (b) is introduced into a distillation step
(d) the reflux ratio of the distillation step is controlled by supplying water to said distillation step.

11. Process according to claim 10 wherein the water has a content of constituents other than water lower than or equal to 12 % by weight relative to the total weight of the water and of such constituents.

12. Process according to claim 11 wherein the constituents other than water, comprise dichloropropanols

13. Process according to claim 1, wherein the reaction is carried out continuously.

14. Process according to anyone of claims 1 to 13, wherein the glycerol used has at least partially been produced from renewable raw materials

15. Process for separation of a mixture containing at least water, dichloropropanol and hydrogen chloride wherein the mixture is separated in a distillation step wherein the sum of materials fed to said distillation step has a hydrogen chloride concentration which is lower than the hydrogen chloride concentration in the binary azeotropic composition hydrogen chloride/water at the pressure of the distillation.
